# EUROPÄISCHE PATENTSCHRIFT

(11) **EP 0 786 235 B1**
(45) Veröffentlichungstag und Bekanntmachung des Hinweises auf die Patenterteilung: **05.04.2000**
(21) Anmeldenummer: 97100775.2
(22) Anmeldetag: 20.01.1997
(51) Int. Cl.: A61B 17/70, A61B 17/58

(54) **Implantat für die interne Fixierung von Wirbelköpern, und Werkzeug zum Abschneiden von Pedikelschrauben**
Implant for internal fixation of vertebrae, and pedicle screw cutter
Implant pour fixation interne des vertèbres, et outil de coupe des vis pédiculaires

(30) Priorität: 24.01.1996 CH 18696
(43) Veröffentlichungstag der Anmeldung: 30.07.1997
(60) Teilanmeldung: 99106720.8
(73) Patentinhaber: Hermann, Werner, CH-6312 Steinhausen (CH)
(72) Erfinder: Hermann, Werner, 6312 Steinhausen (CH); Matzen, Klaus A., 86199 Augsburg-Göggingen (DE)
(74) Vertreter: Hotz, Klaus, Dipl.-El.-Ing./ETH Patentanwalt

(56) Entgegenhaltungen:
- EP-A- 0 346 521
- EP-A- 0 384 001
- WO-A-93/15697
- US-A- 4 987 892

## Beschreibung

Die Erfindung betrifft ein Implantat für die interne Fixierung von Wirbelkörpern nach dem Oberbegriff des Anspruchs **1**.

Derartige Implantate finden bei krankhafter Veränderung (Spondylose) an Wirbelkörpern und Bandscheiben Anwendung, wobei die Stellung der Wirbelkörper korrigiert und die durch die Korrektur hergestellte relative Lage der betroffenen Wirbelkörper fixiert wird.

Aus einem Datenblatt ***Tape Nr. 20098*** der Mediothek der Medizinischen Fakultät der Universität Basel vom 20.1.1988 ist ein Implantat bekannt, das eine Verbindungsstange in Form einer Schraubenspindel aufweist. Die Pedikelschrauben werden von zwei Verbindungsstücken gehalten, die durch Verdrehen verstellbar auf der Schraubenspindel angeordnet sind. Ein Implantat dieser Bauweise kann nur ungenügend an die Krümmung der Wirbelsäule angepasst werden.

Ein mit der ***US-Patentschrift Nr. 5,409,488*** bekannt gewordenes Implantat für Spondylose besteht aus zwei Verbindungsstücken, welche in ihrer Distanz zueinander einstellbar verdrehbar auf Schraubenspindeln angeordnet sind. Die Schraubenspindeln sind mittels eines dritten Verbindungsstückes derart miteinander verbunden, dass ihre Längsachsen sich unter einem Winkel von ca. 15° schneiden. Dadurch soll die Anpassung des Implantates an die Krümmung der Wirbelsäule ermöglicht werden. Die Schraubenspindeln sind mit einem Zapfen, der eine Ringnut aufweist im dritten Verbindungsstück geführt, wobei ein in die Ringnut eingreifender Stift die Schraubenspindeln gegen axiale Verschiebungen sichert. Die Schraubenspindeln weisen einen angeformten Sechskant auf, mittels welchem sie unabhängig voneinander verdreht werden können. In den Verbindungsstücken werden Knochenschrauben gehalten, welche in die Wirbelkörper eingeschraubt sind. Der glatte, gewindelose Schaft der Knochenschrauben wird in einem Halteelement der Verbindungsstücke verdrehbar und in seiner Längsrichtung verschiebbar geführt. Das Halteelement weist ein Klemmglied in Form einer Gewindemuffe auf, durch welche der gewindelose Schaft der Knochenschraube festgeklemmt werden kann. Zu diesem Zweck ist in einer Oeffnung der Verbindungsstücke ein zweiteiliges Einsatzteil angeordnet, das in dem einen Teil ein Innengewinde für die Gewindemuffe aufweist. Im anderen Teil ist anschliessend an das Innengewinde eine konisch verlaufende Partie einer Führungsbohrung der Knochenschraube vorgesehen, in welcher ein mit einem Längsschlitz versehener konischer Klemmring angeordnet ist. Beim Anziehen der Gewindemuffe wird der sowohl in der Gewindemuffe als auch im konischen Klemmring und im Einsatzteil geführte gewindelose Schaft der Knochenschraube mittels des konischen Klemmringes festgeklemmt. Gleichzeitig werden die beiden Teile des Einsatzteiles gegen die Innenwände der Oeffnung der Verbindungsstücke gepresst. Um eine einwandfreie sichere Festhaltung der Knochenschrauben zu erreichen ist das zweiteilige Einsatzteil an seinen Enden mit Ringnuten versehen, in welchen leicht vorstehend geschlitzte Federringe angeordnet sind, die sich beim Anziehen der Gewindemuffe einerseits im Verbindungsstück und andererseits in den Ringnuten des Einsatzteiles festkrallen. Mit vorstehend beschriebenem Implantat soll eine bessere Anpassung an die Krümmung der Wirbelsäule erzielt werden. Da die Anpassung jedoch hauptsächlich auf die vorgegebene relative Lage der Schraubenspindeln zueinander beruht, ist letztendlich eine Anpassung an individuelle Gegebenheiten kaum möglich.

Aus der ***WO-A-93/15697*** ist ebenfalls ein Implantat für die interne Fixierung von Wirbelkörpern bekannt, wobei Pedikelschrauben vorgesehen sind, die in den Wirbelkörpern befestigt und über mindestens eine Verbindungsstange und Verbindungsstücke miteinander bekannt sind. Die Verbindungsstange ist der individuellen Krümmung der Wirbelsäule entsprechend gebogen. Die Verbindungsstücke weisen eine Klemmschraube auf, in welcher eine Bohrung für die Führung und Festhaltung der Verbindungsstange vorgesehen ist.

Aus der ***EP-A-0 384 001*** ist ein weiteres Implantat für die interne Fixierung von Wirbelkörpern bekannt. Bei diesem wird aber die Pedikelschraube gleichzeitig als Klemmschraube verwendet und dient zum Zusammenspannen eines zweiteiligen Klemmkörpers, der die Verbindungsstange in einer sogenannt offenen Klemmung festhält. Die zum Kontakt mit der Verbindungsstange vorgesehene Innenfläche des Klemmkörpers weist zwar eine zylindermantelförmige Ausnehmung auf, womit eine bessere Anlage erreicht werden soll. Die restliche Anlagefläche besteht aus zwei zylindermantelförmigen Bereichen.

Der Erfindung liegt daher die Aufgabe zugrunde, ein Implantat der eingangs genannten Art vorzuschlagen, das eine bessere Anpassung der Bohrung an die entsprechend der individuelle Krümmungen der Wirbelsäule gebogene Verbindungsstange bei gleichzeitig einwandfreier Klemmung ermöglicht.

Diese Aufgabe wird durch die Anspruch 1 angegebene Erfindung gelöst. Vorteilhafte Weiterbildungen der erfindungsgemässen Anordnung sind durch die Ansprüche **2** bis **6** definiert.

Die die Pedikelschrauben über die Verbindungsstücke miteinander verbindende Verbindungsstange wird der individuellen Krümmung der Wirbelsäule entsprechend gebogen. In den Verbindungsstücken sind Klemmschrauben angeordnet, in welchen eine Bohrung für die Führung und Festhaltung der Verbindungsstange vorgesehen ist. Die Oberfläche der Bohrung ist nach aussen gewölbt, wobei der Durchmesser der Bohrung an ihren Enden annähernd dem Durchmesser der Verbindungsstange entspricht.

Die mit der Erfindung erzielten Vorteile sind darin zu sehen, dass eine individuelle Anpassung des Implantats an die Krümmung der Wirbelsäule ermöglicht wird. Dies wird insbesondere durch die entsprechend geformte Verbindungsstange ermöglicht, die in der Bohrung der Klemmschraube lediglich an zwei Stellen linienförmig aufliegend gehalten wird, wobei die Bohrung derart gestaltet werden kann, dass Verbindungsstangen verschiedenster Biegungen befestigt werden können. Vorteilhaft sind der einfache und daher billige Aufbau, wobei insbesondere keine Schraubenspindeln benötigt werden und die Klemmung und Festhaltung einer Pedikelschraube und der Verbindungsstange nur mit einer Klemmschraube bewerkstelligt werden kann.

Im folgenden wird die Erfindung anhand mehrerer Ausführungsbeispiele im Zusammenhang mit der Zeichnung näher erläutert. Es zeigen:

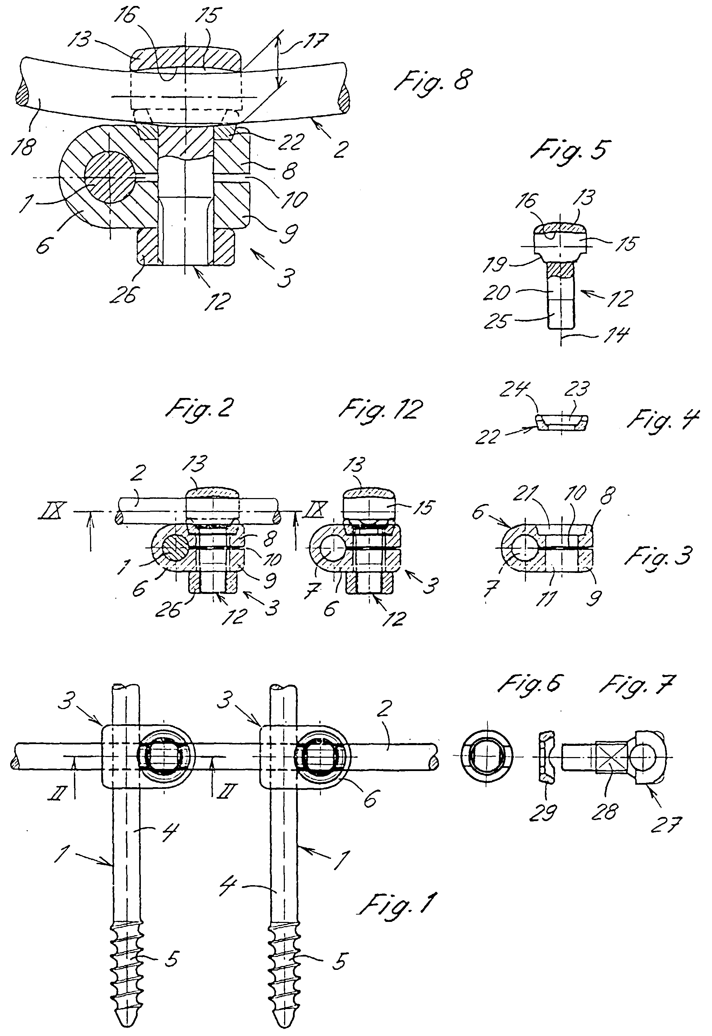

- **Fig. 1**: eine Seitenansicht des erfindungsgemässen Implantats mit zwei Pedikelschrauben,
- **Fig. 2**: einen Schnitt gemäss der Linie II-II in der Fig.1,
- **Fig. 3**: einen Schnitt durch einen Klemmkörper eines Verbindungsstückes des Implantats,

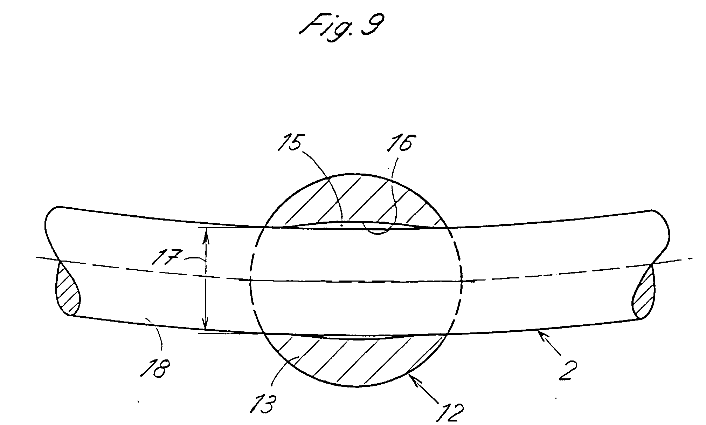

- **Fig. 4**: einen Schnitt durch eine Spannscheibe des Verbindungsstückes,
- **Fig. 5**: einen Teilschnitt durch eine Klemmschraube des Verbindungsstückes,
- **Fig. 6**: einen Schnitt und eine Draufsicht durch beziehungsweise auf die Spannscheibe des Verbindungsstückes in einer anderen Ausführung,
- **Fig. 7**: eine Ansicht der Klemmschraube des Verbindungsstückes in einer anderen Ausführung,
- **Fig. 8**: einen Schnitt durch ein Verbindungsstück mit einer gebogenen Verbindungsstange in grösserem Massstab,
- **Fig. 9**: einen Schnitt gemäss der Linie IX-IX in der Fig.2 in grösserem Massstab,
- **Fig. 10**: eine Rückansicht eines Teiles einer Wirbelsäule mit zwei Implantaten für vier Wirbelkörper,
- **Fig.11**: eine Seitenansicht eines Teiles einer Wirbelsäule mit den Implantaten gemäss Fig.10 und einem Werkzeug zum Abschneiden der Pedikelschrauben, und
- **Fig. 12**: ein weiterer Schnitt durch ein Verbindungsstück.

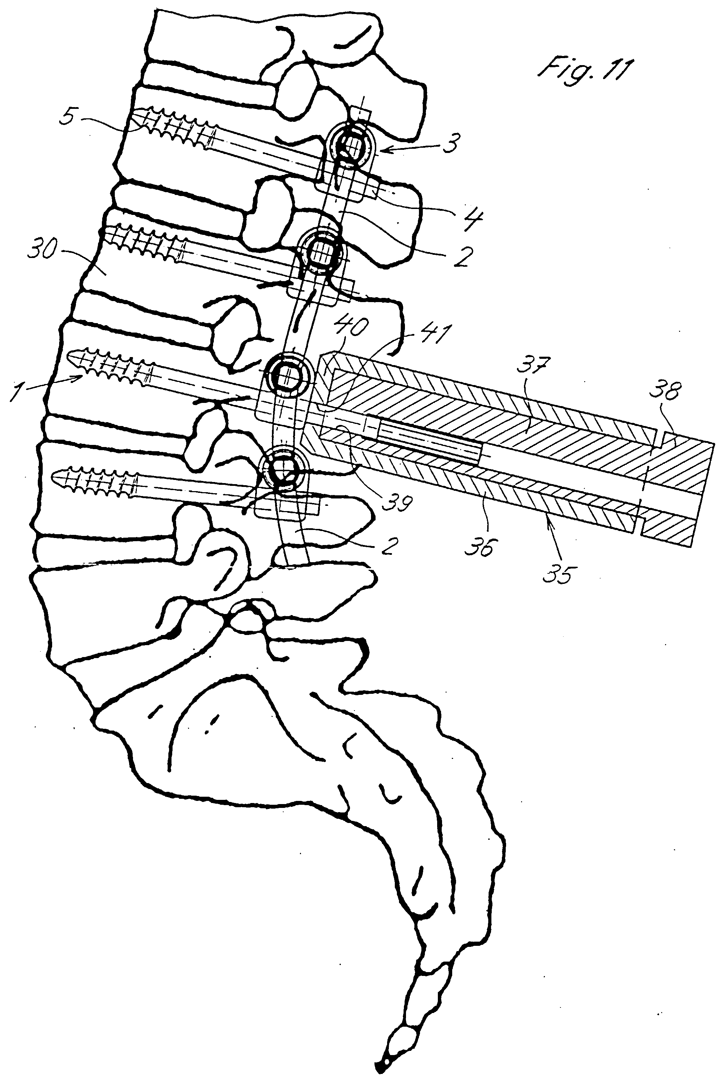

In den Figuren sind mit **1** Pedikelschrauben bezeichnet, die über eine Verbindungsstange **2** und Verbindungsstücke **3** miteinander verbunden sind. Die Pedikelschrauben weisen einen Schaft **4** und ein Gewindeteil **5** auf, mit welchem sie in Wirbelkörper **30 (Fig.10,11)** einschraubbar sind. Die Verbindungsstücke **3** bestehen aus einem Klemmkörper **6**, der eine erste Bohrung **7** für die Aufnahme einer Pedikelschraube **1** und einen mit der ersten Bohrung **7** verbundenen, zwei federnde Spannbacken **8,9** bildenden Schlitz **10** aufweist. In den Spannbacken **8,9** ist eine die erste Bohrung **7** kreuzende, senkrecht zum Schlitz **10** verlaufende zweite Bohrung **11** für die Aufnahme einer Klemmschraube **12** des Verbindungsstückes **3** vorgesehen. In einem Kopf **13** der Klemmschraube **12** ist eine senkrecht zur Längsachse **14** der Klemmschraube **12** verlaufende Bohrung **15** für die Führung und Festhaltung der Verbindungsstange **2** angeordnet. Die Oberfläche **16** der Bohrung **15** ist nach innen und nach aussen gewölbt, wobei der Durchmesser **17** der Bohrung **15** an ihren Enden annähernd dem Durchmesser der Verbindungsstange **2** entspricht und die Wölbung beispielsweise kreis-oder ellipsenförmig ist. In der derartig gestalteten Bohrung **15** kann sowohl ein gerader als auch ein gebogener Teil **18** einer individuell an die Krümmung der Wirbelsäule angepassten Verbindungsstange **2** geführt und gehalten werden, wobei die Verbindungsstange **2** an beiden Enden der Bohrung **15** linienförmig aufliegt **(Fig.8,9).** Ein Uebergang **19** zwischen dem **Kopf 13** und einem Schaft **20** der Klemmschraube **12** ist konisch ausgebildet, wobei die Bohrung **15** teilweise im konischen Uebergang **19** verläuft.

In der einen Spannbacke **8** des Klemmkörpers **6** ist konzentrisch zur zweiten Bohrung **11** eine konische Vertiefung **21** vorgesehen, in welcher eine entsprechend geformte Spannscheibe **22** angeordnet ist. Die Spannscheibe **22** weist eine dem konischen Uebergang **19** der Klemmschraube **12** angepasste konische Vertiefung **23** auf, wobei ein Rand **24** gebildet wird, in welchem ein Teil der Bohrung **15** verläuft.

Beim Fixieren des Implantats werden durch Verschrauben eines Gewindes **25** der Klemmschraube **12** mit einer Mutter **26** sowohl die Pedikelschrauben **1** als auch die Verbindungsstange **2** gleichzeitig festgeklemmt.

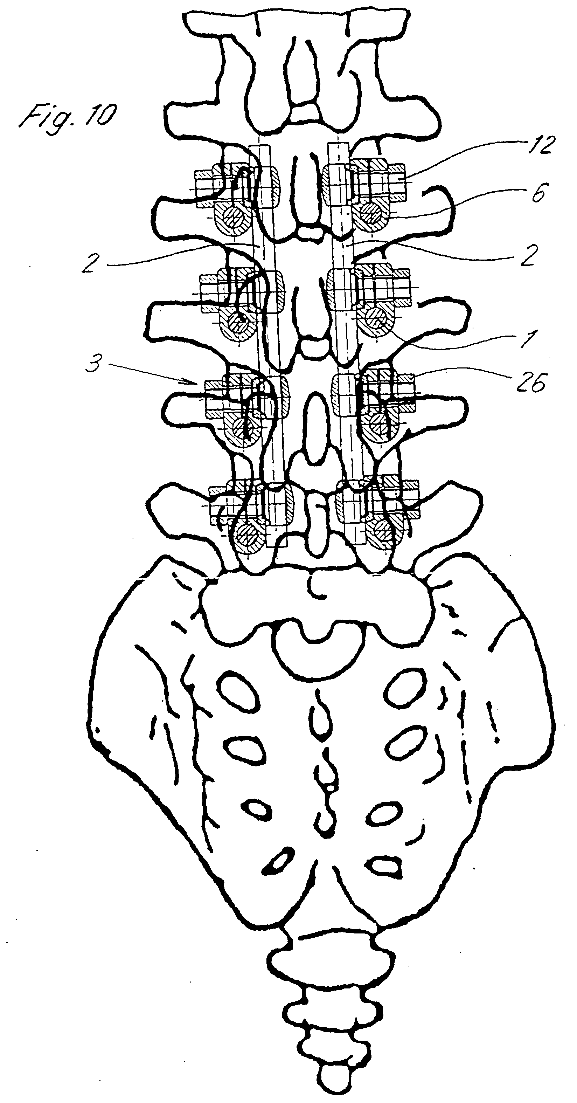

Gemäss der Ausführung nach **Fig.6** und **7** weist die Klemmschraube **27** zwei parallel verlaufende Abflachungen **28** auf, die mit entsprechend geformten, nicht näher dargestellten Abflachungen in der Spannscheibe (mit Langloch passend zu Abflachung **28) 29** und der zweiten Bohrung **11** korrespondieren. Mit dieser Massnahme soll ein Verdrehen der Klemmschraube **27** verhindert werden beziehungsweise in gelöstem Zustand das Mitdrehen der Spannscheibe garantieren, damit in jeder Lage sichergestellt ist, dass die Verbindungstange nicht aus den Vertiefungen rutscht und damit in der Lage wäre sich zu Verdrehen.

Nach **Fig.11** besteht ein Werkzeug **35** zum Abschneiden der Pedikelschrauben **1** auf die erforderliche Länge aus einem Zylinder **36,** in welchem ein Drehkolben **37** geführt ist. Der Drehkolben **37** weist einen aus dem Zylinder **36** herausragenden Kopf **38** und eine in seiner Längsrichtung verlaufende exzentrische Bohrung **39** auf, die mit einer in einem Boden **40** des Zylinders **36** befindlichen weiteren exzentrischen Bohrung **41** fluchtet. Die beiden Bohrungen **39,41** sind für die Aufnahme des Schaftes **4** der Pedikelschraube **1** bestimmt. Nach der definitiven Fixierung des Implantates wird das Werkzeug **35** bis zum Verbindungsstück **3** auf den Schaft **4** aufgeschoben und dieser durch Verdrehen des Kopfes **38** bzw. des Drehkolbens **37** abgeschnitten.

### Bezugszeichenliste

- **1**: Pedikelschrauben
- **2**: Verbindungsstange
- **3**: Verbindungsstücke
- **4**: Schaft
- **5**: Gewindeteil
- **6**: Klemmkörper
- **7**: Erste Bohrung
- **8**: Spannbacke
- **9**: Spannbacke
- **10**: Schlitz
- **11**: Zweite Bohrung
- **12**: Klemmschraube
- **13**: Kopf
- **14**: Längsachse
- **15**: Bohrung
- **16**: Oberfläche
- **17**: Durchmesser
- **18**: Gebogener Teil
- **19**: Konischer Uebergang
- **20**: Schaft
- **21**: Konische Vertiefung
- **22**: Spannscheibe
- **23**: Konische Vertiefung
- **24**: Rand
- **25**: Gewinde
- **26**: Mutter
- **27**: Klemmschraube
- **28**: Abflachungen
- **29**: Spannscheibe mit Langloch
- **30**: Wirbelkörper
- **35**: Werkzeug
- **36**: Zylinder
- **37**: Drehkolben
- **38**: Kopf
- **39**: Bohrung
- **40**: Boden
- **41**: Weitere Bohrung

## Patentansprüche

1. Implantat für die interne Fixierung von Wirbelkörpern, wobei Pedikelschrauben (**1**) vorgesehen sind, die in den Wirbelkörpern (**30**) befestigbar und über Verbindungsstücke (**3**) und mindestens eine Verbindungsstange (**2**) miteinander verbunden sind,
welche Verbindungsstange (**2**) der individuellen Krümmung der Wirbelsäule entsprechend gebogen ist, und wobei
jedes Verbindungsstück (**3**) eine Klemmschraube (**12**) aufweist, in welcher eine Bohrung (**15**) für die Führung und Festhaltung der Verbindungsstange (**2**) vorgesehen ist,
**dadurch gekennzeichnet**,
dass die Oberfläche (**16**) der Bohrung (**15**) nach aussen gewölbt ist, wobei der Durchmesser (**17**) der Bohrung (**15**) an ihren Enden annähernd dem Durchmesser der Verbindungsstange (**2**) entspricht.

2. Implantat nach Anspruch **1**,
**dadurch gekennzeichnet**,
dass die Bohrung (**15**) für die Verbindungsstange (**2**) in einem Kopf (**13**) der Klemmschraube (**12**) angeordnet ist und senkrecht zur Längsachse (**14**) der Klemmschraube (**12**) verläuft.

3. Implantat nach Anspruch **2**,
**dadurch gekennzeichnet**,
dass die Oberfläche (**16**) der Bohrung (**15**) für die Verbindungsstange (**2**) kreis-oder ellipsenförmig gewölbt ist.

4. Implantat nach Anspruch **1**,
**dadurch gekennzeichnet**,
dass ein Klemmkörper (**6**) der Verbindungsstücke (**3**) eine erste Bohrung (**7**) für die Aufnahme einer Pedikelschraube (**1**) und einen mit der ersten Bohrung (**7**) verbundenen, zwei federnde Spannbacken (**8,9**) bildenden Schlitz (**10**) auf weist, wobei in den Spannbacken (**8,9**) eine die erste Bohrung (**7**) kreuzende, senkrecht zum Schlitz (**10**) verlaufende zweite Bohrung (**11**) für die Aufnahme der Klemmschraube (**12**) vorgesehen ist.

5. Implantat nach Anspruch **4**,
**dadurch gekennzeichnet**,
dass die Klemmschraube (**27**) zwei parallel zueinander verlaufende Abflachungen (**28**) aufweist.

6. Implantat nach Anspruch **5**,
**dadurch gekennzeichnet**,
dass die zweite Bohrung (**11**) eine mit den Abflachungen (**28**) der Klemmschraube (**27**) korrespondierende Ausformung aufweist.

## Claims

1. Implant for internal fixation of vertebrae, there being provided pedicle screws (1) which are adapted to be fastened in the vertebrae (30) and are joined to one another via connecting pieces (3) and at least one connecting rod (2),
said connecting rod (2) having been bent to suit the individual curvature of the spine, and
each connecting piece (3) having a clamping screw (12) in which there is provided a bore (15) for guiding and holding the connecting rod (2),
characterised in that
the surface (16) of the bore (15) is vaulted outwards, with the diameter (17) of the bore (15) being approximately the same at its ends as the diameter of the connecting rod (2).

2. Implant according to claim 1,
characterised in that
the bore (15) for the connecting rod (2) is disposed in a head (13) of the clamping screw (12) and runs perpendicular to the longitudinal axis (14) of the clamping screw (12).

3. Implant according to claim 2,
characterised in that
the surface (16) of the bore (15) for the connecting rod (2) is vaulted in the shape of a circle or ellipse.

4. Implant according to claim 1,
characterised in that
a clamping body (6) of the connecting pieces (3) has a first bore (7) for accommodating a pedicle screw (1) and a slot (10) which is connected to the first bore (7) and forms two resilient clamping jaws (8, 9), there being provided in the clamping jaws (8, 9) a second bore (11) which crosses the first bore (7) and runs perpendicular to the slot (10) in order to accommodate the clamping screw (12).

5. Implant according to claim 4,
characterised in that
the clamping screw (27) has two flattened areas (28) running parallel to one another.

6. Implant according to claim 5,
characterised in that
the second bore (11) has a hollowed-out area which corresponds to the flattened areas (28) of the clamping screw (27).

## Revendications

1. Implant pour la fixation interne de vertèbres, dans lequel on prévoir des vis pédiculaires (1), qui peuvent être fixées dans les vertèbres (3) et sont reliées entre elles par l'intermédiaire d'éléments de liaison (3) et d'au moins une tige de liaison (2),
laquelle tige de liaison (2) est coudée conformément à la courbure individuelle de la colonne vertébrale, et dans lequel
chaque élément de liaison (3) comporte une vis de blocage (12), dans laquelle un perçage (15) est prévu pour le guidage et la fixation de la tige de liaison (2),
caractérisé en ce
que la surface (16) du perçage (15) est cintrée vers l'extérieur, le diamètre (17) du perçage (15) au niveau des extrémités de la surface correspondant approximativement au diamètre de la tige de liaison (2).

2. Implant selon la revendication 1, caractérisé en ce
que le perçage (15) pour la tige de liaison (2) est disposé dans un corps (13) de la vis de blocage (2) et s'étend perpendiculairement à l'axe longitudinal (14) de la vis de blocage (12).

3. Implant selon la revendication 2, caractérisé en ce
que la surface (16) du perçage (15) pour la tige de liaison (2) est cintrée avec une forme circulaire ou elliptique.

4. Implant selon la revendication 1, caractérisé en ce
qu'un corps de blocage (6) des éléments de liaison (3) comporte un premier perçage (7) destiné à loger une vis pédiculaire (1), et une fente (10), qui est reliée au premier perçage (7) et forme deux mâchoires élastiques de serrage (8,9), un second perçage (11), qui croise le perçage (13) et est perpendiculaire à la fente (10) et sert à loger la vis de blocage (12), étant prévu dans les mâchoires de serrage (8,9).

5. Implant selon la revendication 4, caractérisé en ce
que la vis de blocage (27) possède deux méplats (28) qui sont parallèles entre eux.

6. Implant selon la revendication 5, caractérisé en ce
que le second perçage (11) comporte une conformation correspondant aux méplats (28) de la vis de blocage (27).
